# EUROPEAN PATENT APPLICATION

(11) **EP 1 197 147 A1**
(43) Date of publication of application: **17.04.2002**
(21) Application number: 00203559.0
(22) Date of filing: 13.10.2000
(51) Int. Cl.: A01N 1/00, A61K 35/14

(54) **Method of inactivating viral particles in a blood product**

(71) Applicant: Boston Clinics PDT B.V., 2333 AL Leiden (NL)
(72) Inventor: Dubbelman, Thomas Marinus Albert Remko, 2406 JJ Alphen a/d Rijn (NL); Lagerberg, Johannes Wilhelminus Maria, 2317 DV Leiden (NL)
(74) Representative: Van Breda, Jacobus (NL)

(57) **Abstract**

The present invention relates to a method of inactivating viral particles in a blood product, more in particular red blood cells and platelets, comprising the steps of
- combining said blood product with a photosensitiser, and
- activating the photosensitiser.
In accordance with the present invention, a positively-charged sensitizer is used having a defined structure. Surprisingly, the damage to the blood product is reduced.

## Description

The present invention relates to a method of inactivating viral particles in a blood product comprising the steps of
- combining said blood product with a photosensitiser, and
- activating the photosensitiser.

The transmission of viruses (HIV, hepatitis B and C) by transfusion with infected blood products presents a serious health problem.

US 5,360,734 describes a method of inactivating viral pathogens in a body fluid, such as plasma, red cells, platelets, leukocytes and bone marrow.

The method comprises adding the photosensitiser to the blood product and irradiation with light to activate the photosensitiser, thereby inactivating viral pathogens. The method disclosed in US 5,360,734 is in particular aimed at and reducing the influence of plasma proteins in order to increase the stability of the red cells.

The object of the present invention is to provide a further method of improving the stability of a blood product and in particular red cells and platelets, which method may or may not be used in conjunction with the improvement disclosed in US 5,360,734.

Thus, the present invention relates to a method according to the preamble characterized in that as the photosensitiser a compound is used chosen from the group consisting of compounds with the formula's Ia-d 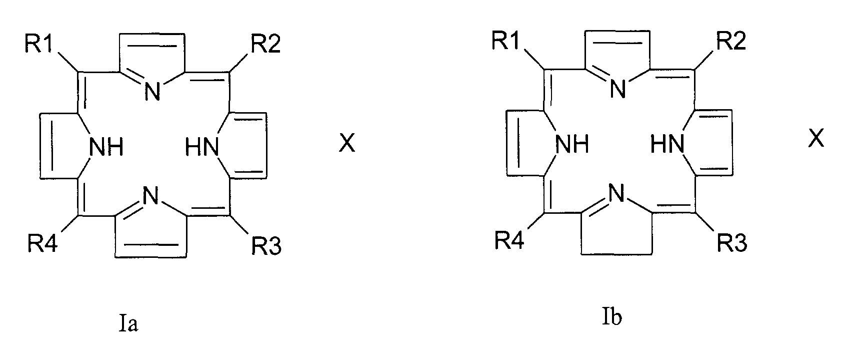 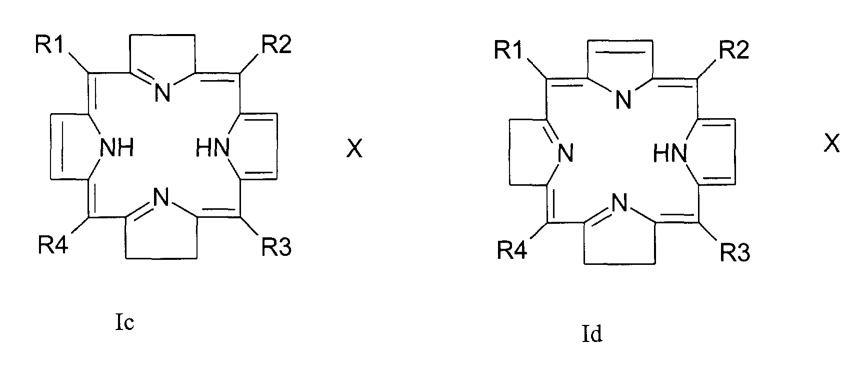 wherein R₁, R₂, R₃ and R₄ are independently chosen from the group consisting of
- hydrogen,
- a halogen atom,
- (C₁-C₂₀)alkyl, (C₁-C₂₀)alkoxy, (C₁-C₂₀)acyl, (C₁-C₂₀)acyloxy, (C₂-C₂₀)alkenyl, or (C₂-C₂₀)alkynyl, each of which may be linear or branched and each of which may be substituted with one or more groups chosen from
   - hydroxyl,
   - amino which may be substituted with 1 to 3 groups chosen from (C₁-C₂₀)alkyl, (C₂-C₂₀)alkenyl, (C₁-C₂₀)alkoxy, (C₂-C₂₀)alkynyl, and -(R₅-Z)ₘ-R₆ where R₅ is (CH₂)ₙ, Z is O or S, and R₆ is (C₁-C₂₀)alkyl and m and n are, independently, 1-10, each substituent group of the amino group may be linear or branched and each of these may be substituted with one or more groups chosen from hydroxyl, and a halogen atom,
   - nitril, and
   - a halogen atom,
- (C₆-C₂₀)aryl, and (C₆-C₂₀) heterocyclic aryl group each of which may be substituted with one or more groups chosen from
   - hydroxyl,
   - amino which may be substituted with 1 to 3 groups chosen from (C₁-C₂₀)alkyl, (C₂-C₂₀)alkenyl, (C₁-C₂₀)alkoxy, and (C₂-C₂₀)alkynyl, each of which may be linear or branched and each of which may be substituted with one or more groups chosen from hydroxyl, and a halogen atom,
   - nitril,
   - a halogen atom, and
   - (C₁-C₁₀)alkyl, (C₁-C₁₀)alkoxy, (C₂-C₁₀)alkenyl,
   the heterocyclic aryl group containing at least one atom chosen from N, O, P, and S where P, N or S may be substituted with a group chosen from (C₁-C₂₀)alkyl, (C₂-C₂₀)alkenyl, (C₁-C₂₀)alkoxy, and (C₂-C₂₀)alkynyl, each of which may be linear or branched and each of which may be substituted with one or more groups chosen from hydroxyl, and a halogen atom,
at least one of the groups R₁, R₂, R₃ and R₄ contains a quaternary nitrogen atom, and
wherein X is a pharmaceutically acceptable counterion.

Surprisingly, it has been found that by using a photosensitising compound as disclosed above, the damage to the blood product is reduced. This is evident from experiments which are generally recognized as models to determine the extend of damage to a blood product. In particular this was shown in experiments where the hemolysis and potassium leakage are measured.

In the present invention, the term "blood product" is to be understood as any solution comprising at least one of i) red blood cells and ii) platelets. Other cell types may be present and the solution, while being an aqueous solution, may contain proteinaceous components, salts, stabilizers, anti-coagulation agents such as citric acid or heparin, as is generally recognized in the art.

The term "photosensitizer" is, as well recognized in the art, a substance which absorbs light energy as a result of which the photosensitizer is activated. The activated photosensitizer can subsequently react with other compounds. This may result in the photosensitizer being modified or inactivated, but more likely the photosensitizer will return to its original state (before it was activated with light), so as to form a photocatalytic cycle in which the photosensitizer can be used again. In effect, the light energy absorbed is used for the inactivation of viral particles.

The term "viral particle" is understood to mean any RNA or DNA virus, single- or double-stranded and with a membrane or proteinaceous coat that may occur in a blood product. Examples are HIV and hepatitis B.

The term "pharmaceutically acceptable counterion" is understood to be any inorganic or organic negatively charged counterion such as OH⁻, Cl⁻, acetate or citrate. It goes without saying that there are as many counterions X as needed to neutralise the positive charge of the actual active compound I.

Ben-Hur E. et al disclose in Transfusion 35(5), pp401-6 (1999) a method of inactivating virus present in a solution comprising red blood cells. To protect the red blood cells, a protective agent has to be added. As a result of this, which applicants have not been able to replicate due to the difficulties of dissolving the protecting agent Trolox, hemolysis was over 1% after 10 days of storage. In contrast, the method according to the present invention results in 1.5% hemolysis after 5 weeks in the absence of a protecting agent. Of course, the method according to the present invention still allows for the addition of protective agents, such as disclosed in PCT/NL99/00387.

According to a first embodiment at least one of R₁, R₂, R₃ and R₄ is a (C₆-C₂₀) heterocyclic aryl group comprising a nitrogen atom substituted with a group chosen from (C₁-C₂₀)alkyl, (C₂-C₂₀)alkenyl, (C₁-C₂₀)alkoxy, and (C₂-C₂₀)alkynyl, each of which may be linear or branched and each of which may be substituted with one or more groups chosen from hydroxyl, and a halogen atom. Preferably, the heterocyclic aryl group is a pyridinium group, the nitrogen of which is substituted with a (C₁-C₄) alkyl group.

According to a second embodiment at least one of R₁, R₂, R₃ and R₄ is a (C₆-C₂₀) aryl group substituted with an amino which may be substituted with 1 to 3 groups chosen from (C₁-C₂₀)alkyl, (C₂-C₂₀)alkenyl, (C₁-C₂₀)alkoxy, and (C₂-C₂₀)alkynyl, each of which may be linear or branched and each of which may be substituted with one or more groups chosen from hydroxyl, and a halogen atom. Preferably the aryl group is a trialkyl aminophenyl group where alkyl is independently (C₁-C₃) alkyl.

It has been found that quaternary nitrogen atoms in groups as defined above are very suitable for eliminating viral particles while maintaining the integrity of the blood product.

Preferably at least two of R₁, R₂, R₃ and R₄ comprise a quaternary nitrogen atom, and more preferably three of R₁, R₂, R₃ and R₄ comprise a quaternary nitrogen atom. Such compounds appear to give the best result.

The invention will now be elucidated with reference to the exemplary embodiments and the drawing, which shows the degree of hemolysis using a compound in accordance with the present invention (TriP4), and two control compounds.

### EXAMPLES

### Blood product

The blood product used throughout these studies were standard blood cell concentrates (RBCC) without buffy coat, and were provided by the Sanquin Blood Bank, Leiden-Haaglanden, Leiden, the Netherlands, and obtained from healthy human volunteers. Blood from these donors (500 ± 50 ml) was collected in 73 ml citrate-phosphate-dextrose (CPD) in quadruple polyvinylchloride bags (NPBI, Emmer-Compascuum, the Netherlands). RBCCs were prepared as described before (Novotny, 1992). The hematocrit was adjusted to 56 ± 4 with the preservative saline-adenine-glucose-mannitol (SAG-M). SAG-M contains 150 mM NaCl, 50 mM glucose, 29 mM mannitol and 1.25 mM adenine. The number of white cells was 1.8 + 0.9*10⁹ cells/l, as determined on an automatic cell counter (Sysmex K1000, TOA Medical Electronics, Kobe, Japan).

### Photodynamic treatment of virus containing RBCC

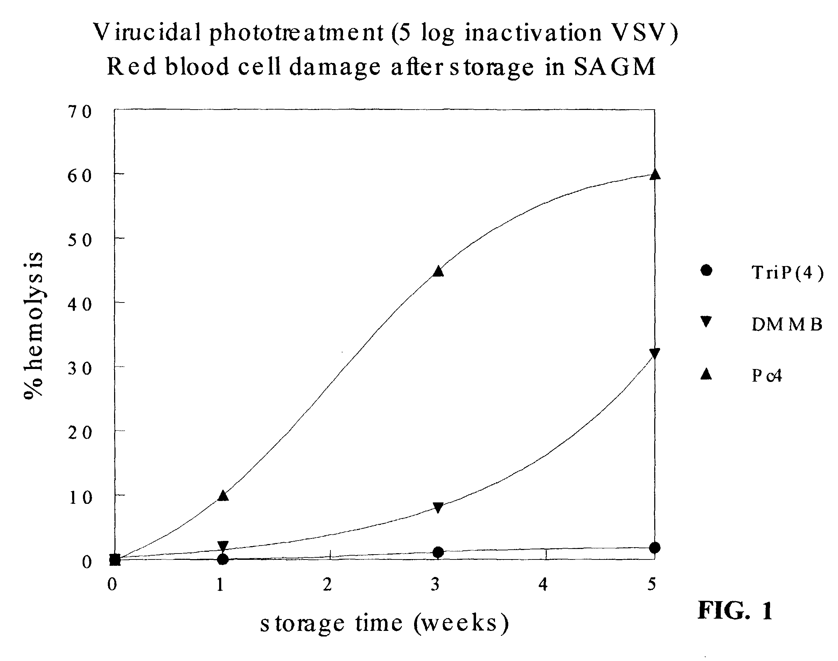

Stock solutions of vesicular stomatitis virus (VSV) (San Juan strain, kindly provided by the Department Virology, Leiden University Medical Center) were added to RBCC such that the volume of the spike was <10% of the total volume of RBCC, and the number of virus particles was about 10⁵/ml. Sensitizers were added to a final concentration of 5 µM aluminium phthalocyanine tetrasulfonate (AIPcS₄, Porphyrin Products, Logan, Utah, USA), 0.45 µM silicon phthalocyanine, HO-SiPc(CH₃)₂(CH₂)₃N(CH₃)₂, (Pc4, donated by Dr. M.E. Kenney, Case Western Reserve University, Cleveland Ohio, USA), 15 µM dimethylmethylene blue (DMMB, ICN Biomedicals BV, Zoetermeer, the Netherlands), and, in accordance with the invention, 25 µM monophenyl-tri(N-methyl-4pyridyl)porphyrin chloride (TriP(4), Mid-Century, Posen, Illinois, USA). The suspensions were thoroughly mixed and divided into 6-ml aliquots in polystyrene culture dishes with a diameter of 9 cm (Greiner, Alphen a/d Rijn, the Netherlands), and agitated at room temperature on a horizontal reciprocal shaker (60 cycles/min, GFL, Burgwedel, Germany) for 5 min. in the dark. The dishes (one dish per time point) were illuminated from above with a 300 W halogen lamp (Philips, Eindhoven, the Netherlands). The light passed through a 1-cm water filter, to avoid heating of the samples. A cut-off filter, only transmitting light with wavelengths above 600 nm, was used in all experiments. The irradiance at the cell layer was 100 W/m², as measured with an IL1400A photometer equipped with a SEL033 detector (International Light, Newburyport, MA, USA). The damage to the red blood cells was established by measuring the degree of hemolysis over a period of 5 weeks. To compare the results with those of Ben-Hur (supra), the solution was illuminated to achieve a 100,000 fold decrease in viral load. As is shown in Fig. 1, the method according to the present invention achieves a better result after 10 days than the method of Ben-Hur, even in the absence of a protecting agent.

## Claims

1. Method of inactivating viral particles present in a blood product comprising the steps of
- combining said blood product with a photosensitiser, and
- activating the photosensitiser,
**characterized, in that** as the photosensitiser a compound is used chosen from the group consisting of compounds with the formulas Ia-Id 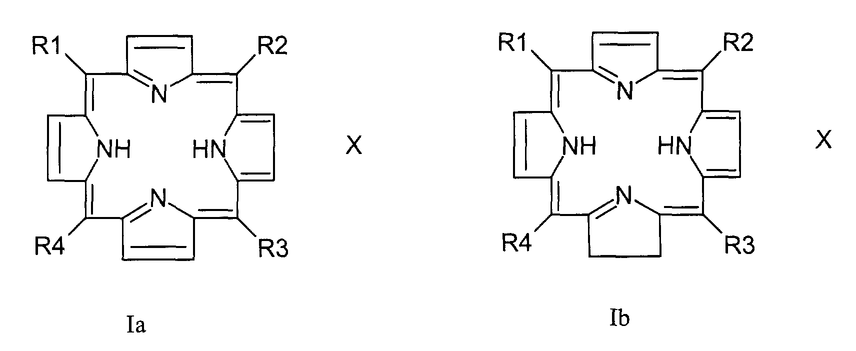 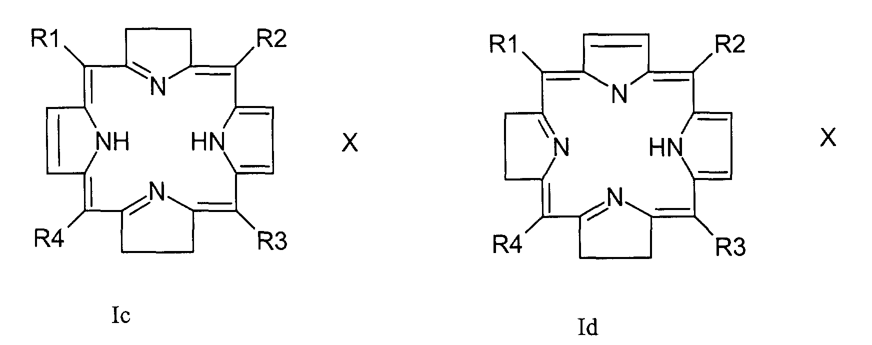 wherein R₁, R₂, R₃ and R₄ are independently chosen from the group consisting of
- hydrogen,
- a halogen atom,
- (C₁-C₂₀)alkyl, (C₁-C₂₀)alkoxy, (C₁-C₂₀)acyl, (C₁-C₂₀)acyloxy, (C₂-C₂₀)alkenyl, or (C₂-C₂₀)alkynyl, each of which may be linear or branched and each of which may be substituted with one or more groups chosen from
- hydroxyl,
- amino which may be substituted with 1 to 3 groups chosen from (C₁-C₂₀)alkyl, (C₂-C₂₀)alkenyl, (C₁-C₂₀)alkoxy, (C₂-C₂₀)alkynyl, and -(R₅-Z)ₘ-R₆ where R₅ is (CH₂)ₙ, Z is O or S, and R₆ is (C₁-C₂₀)alkyl and m and n are, independently, 1-10, each substituent group of the amino group may be linear or branched and each of these may be substituted with one or more groups chosen from hydroxyl, and a halogen atom,
- nitril, and
- a halogen atom,
- (C₆-C₂₀)aryl, and (C₆-C₂₀) heterocyclic aryl group each of which may be substituted with one or more groups chosen from
- hydroxyl,
- amino which may be substituted with 1 to 3 groups chosen from (C₁-C₂₀)alkyl, (C₂-C₂₀)alkenyl, (C₁-C₂₀)alkoxy, and (C₂-C₂₀)alkynyl, each of which may be linear or branched and each of which may be substituted with one or more groups chosen from hydroxyl, and a halogen atom,
- nitril,
- a halogen atom, and
- (C₁-C₁₀)alkyl, (C₁-C₁₀)alkoxy, (C₂-C₁₀)alkenyl,
the heterocyclic aryl group containing at least one atom chosen from N, O, P, and S where P, N or S may be substituted with a group chosen from (C₁-C₂₀)alkyl, (C₂-C₂₀)alkenyl, (C₁-C₂₀)alkoxy, and (C₂-C₂₀)alkynyl, each of which may be linear or branched and each of which may be substituted with one or more groups chosen from hydroxyl, and a halogen atom,
at least one of the groups R₁, R₂, R₃ and R₄ contains a quaternary nitrogen atom, and
wherein X is a pharmaceutically acceptable counterion.

2. Method according to claim 1, **characterized, in that** at least one of R₁, R₂, R₃ and R₄ is a (C₆-C₂₀) heterocyclic aryl group comprising a nitrogen atom substituted with a group chosen from (C₁-C₂₀)alkyl, (C₂-C₂₀)alkenyl, (C₁-C₂₀)alkoxyl, and (C₂-C₂₀)alkynyl, each of which may be linear or branched and each of which may be substituted with one or more groups chosen from hydroxyl, and a halogen atom.

3. Method according to claim 2, **characterized, in that** the heterocyclic aryl group is a pyridinium group, the nitrogen of which is substituted with a (C₁-C₄) alkyl group.

4. Method according to claim 1, **characterized in that** at least one of R₁, R₂, R₃ and R₄ is a (C₆-C₂₀) aryl group substituted with an amino which may be substituted with 1 to 3 groups chosen from (C₁-C₂₀)alkyl, (C₂-C₂₀)alkenyl, (C₁-C₂₀)alkoxy, and (C₂-C₂₀)alkynyl, each of which may be linear or branched and each of which may be substituted with one or more groups chosen from hydroxyl, and a halogen atom.

5. Method according to claim 4, **characterized in that** the aryl group is a trialkyl aminophenyl group where alkyl is independently (C₁-C₃) alkyl.

6. Method according to any of the preceding claims, **characterized in that** at least two of R₁, R₂, R₃ and R₄ comprise a quaternary nitrogen atom.

7. Method according to claim 6, **characterized in that** three of R₁, R₂, R₃ and R₄ comprise a quaternary nitrogen atom.
